# FASCICULE DE BREVET EUROPEEN

(11) **EP 0 311 492 B1**
(45) Date de publication et mention de la délivrance du brevet: **30.03.1994**
(21) Numéro de dépôt: 88402475.3
(22) Date de dépôt: 30.09.1988
(51) Int. Cl.: G01N 33/53, G01N 33/543, G01N 33/577, G01N 33/569, G01N 33/574

(54) **Trousse et méthode de dosage immunométrique applicables à des cellules entières**
Immuntestsatz und -verfahren für ganze Zellen
Kit and immunoassay method applicable to whole cells

(30) Priorité: 30.09.1987 FR 8713537
(43) Date de publication de la demande: 12.04.1989
(73) Titulaire: ELF SANOFI, 75008 Paris (FR)
(72) Inventeur: Carrière, Dominique, F-34160 Castries (FR)
(74) Mandataire: Gillard, Marie-Louise

(56) Documents cités:
- EP-A- 0 101 912
- EP-A- 0 119 736
- EP-A- 0 179 007
- EP-A- 0 204 522
- WO-A-84/03151
- WO-A-86/02733
- WO-A-86/07633
- WO-A-87/05398
- WO-A-87/06620
- US-A- 4 677 061
- DORLAND, "Illustrated Medical Dictionary, 26ème édition, 1981, W.B.Saunders Company, Philadelphia, PA (US); pp. 90-91#
- TRANSPLANTATION, vol. 36, no. 6, 1983, US; R.E. MORRIS, pp. 719-725#

## Description

La présente invention concerne une trousse et une méthode de dosage immunométrique utilisant cette trousse, pour le dosage d'antigènes de surface caractéristiques de populations ou de sous-populations de cellules. La méthode de dosage immunométrique et la trousse correspondante sont également destinées au dosage des cellules elles-mêmes par l'intermédiaire du dosage de leurs antigènes de surface. Ces dosages sont applicables au diagnostic.

La connaissance des antigènes ou marqueurs de la surface cellulaire a fait d'énormes progrès avec la mise au point de l'hybridation lymphocytaire et la découverte des anticorps monoclonaux par KOEHLER et MILSTEIN, (Nature, 1975, 256, 495-497). Les anticorps monoclonaux ont notamment permis la mise en évidence et l'analyse de marqueurs de surface ou antigènes membranaires de cellules d'origines les plus variées. Ces marqueurs (ou antigènes) peuvent être de différentes natures : protéines, glycoprotéines ou glycolipides. Les caractérisations recherchées s'adressent alors principalement à des marqueurs de tissu ou d'organe, à des marqueurs d'états de différenciation ou d'activation de cellules normales et à l'identification ou au typage de cellules normales ou cancéreuses. Un domaine d'application particulièrement important est l'étude des lignées cellulaires de l'hématopoïèse (érythrocytaire, megakaryocytaire, granulocytaire, monocytaire, lymphocytaire).

Ainsi, par exemple, les anticorps monoclonaux ont permis de préciser les caractéristiques de surface respectives des lymphocytes T et B. Les marqueurs correspondants seuls ou en combinaison identifient des stades de différenciation et de spécialisation fonctionnelle des lymphocytes. Par convention internationale, les marqueurs de surface des leucocytes humains ont été classés dans des groupes ou classes de différenciation (CD) définis lors du Sous-Comité IUIS-OMS, 1984 et décrits dans Bulletin de l'Organisation Mondiale de la Santé, 1984, 62 (5), 813-815.

L'identification de ces marqueurs rendue possible grâce aux anticorps monoclonaux a permis d'accéder à leur structure et à leurs fonctions biologiques. Par exemple, les molécules des marqueurs CD4 et CD8 participent à des fonctions d'adhésion leucocytaire et se trouvent à la surface des lymphocytes T à fonction auxiliaire et inductrice (marqueur CD4) ou à fonction cytotoxique et suppressive (marqueur CD8), respectivement.

L'établissement de ces connaissances a permis l'utilisation de ces marqueurs grâce aux anticorps qui les reconnaissent pour le diagnostic et le suivi de pathologies variées dont, notamment, les hémopathies malignes (leucémies, lymphomes, etc.), et les états de dysfonctionnement du système immunitaire (maladies autoimmunes, déficits immunitaires congénitaux ou acquis comme le SIDA, etc.) (BRETON-GORIUS et VAINCHENKER, Le Biologiste, 1987, XXI, n^{o} 167 63-70, SHAW, Immunology today, 1987, 8 (1), 1-3).

Les anticorps monoclonaux sont aujourd'hui des outils irremplaçables de la biologie clinique appliquée aux analyses cellulaires.

Il existe des méthodes de dénombrement de cellules qui utilisent le marquage de leurs antigènes de surface, mais ces méthodes sont souvent longues, laborieuses, difficiles à mettre en oeuvre, et les résultats en sont parfois aléatoires.

Les méthodes connues et utilisées pour mesurer l'expression normale ou modifiée des antigènes de la surface de la cellule peuvent être séparées en deux groupes. Dans le premier groupe, les antigènes sont mesurés à l'aide d'équipements de laboratoire complexes et spécialisés reposant sur la cytométrie de flux (voir notamment PONCELET et Coll., J. Immunol. Methods, 1985, 85, 65-74) ou les techniques de microscopie quantitative (POULTER et Coll. J. Immunol. Methods, 1987, 98, 227-234). La mise en oeuvre de ces méthodes d'évaluation des antigènes de la cellule est basée sur la mesure de signaux apportés par des anticorps anticellules couplés de manière directe ou indirecte à un réactif marqué par des substances fluorescentes (ou fluorochromes) telles que l'isothiocyanate de fluorescéine ou de rhodamine, ou des enzymes telles que la peroxydase ou la phosphatase alcaline. L'emploi de ces réactifs fluorescents ou enzymatiques associés à des étapes appropriées de lavage conduit alors à l'apparition de fluorescences ou de colorations strictement limitées aux membranes cellulaires et ne diffusant pas dans le milieu environnant. L'utilisation courante de ces méthodes en laboratoire reste limitée par la nécessité d'un appareillage spécialisé et coûteux (microscope à fluorescence associé ou non à un analyseur d'image, cryostat, cytomètre de flux). En outre, l'analyse et l'interprétation des immunomarquages des cellules par ces procédés rendent nécessaire la compétence d'un spécialiste en cytologie.

Un deuxième groupe de méthodes de mesure des antigènes est basé sur l'évaluation quantitative des marqueurs de la population cellulaire globale. Ces méthodes permettent la mesure des antigènes par un marquage soit direct, soit indirect qui est alors réalisé le plus souvent en deux, trois ou quatre étapes. Dans tous les cas, le réactif employé lors de la dernière étape du marquage porte une sonde qui est soit de nature isotopique, Iode 125 par exemple, pour un dosage de type immunoradiométrique (BROW et Coll. J. Immunol. Methods, 1979, 31, 201 - STOCKER et HEUSSER J. Immunol. Methods, 1979, 26, 87-95) soit une enzyme pour un dosage de type immunoenzymométrique, le plus souvent la peroxydase, la phosphatase alcaline ou la β-galactosidase, (VAN LEUVEN et Coll., J. Immunol. Methods, 1978, 23, 109-116 - MORRIS Transplantation, 1983, 36 (6), 719 - BAUMGARTEN J. Immunol. Methods, 1986, 94, 91-98).

Les méthodes de ce dernier groupe sont assez incommodes, laborieuses et hasardeuses à appliquer en raison de la nécessité de nombreux lavages et centrifugations du matériel cellulaire ; il est nécessaire parfois de prélever le milieu coloré résultant de la réaction enzymatique en vue de la mesure spectrophotométrique finale ; enfin la fixation chimique des cellules, qui est le plus souvent utilisée, est à l'origine de la destruction irréversible de certains antigènes particulièrement sensibles aux fixateurs chimiques usuels tels que glutaraldéhyde ou méthanol (DROVER et Coll. J. Immunol. Methods, 1986, 90, 275-281).

Il est connu dans la littérature que l'on peut doser un antigène portant plusieurs déterminants antigéniques, c'est-à-dire plusieurs épitopes, en fixant cet antigène par l'un de ses épitopes grâce à un anticorps immobilisé sur un support solide et en liant à un autre épitope de l'antigène un autre anticorps porteur d'un marqueur enzymatique ou radio-isotopique permettant le dosage.

Une telle technique, souvent appelée technique sandwich, est notamment décrite dans les brevets ou demandes de brevets FR 2 487 983, FR 2 500 166, EP 119 736. Aucun de ces documents ne décrit l'application de cette technique à des cellules entières, même si le mot "cellule" est parfois inclus dans la liste des antigènes auxquels s'applique le procédé.

Dans les brevets ci-dessus, les différents antigènes, objets des exemples décrits, sont tous et uniquement des molécules protéiques, solubles dans l'eau et les liquides physiologiques, telles que hormones, enzymes ou marqueurs tumoraux circulants. Par opposition, il est clair qu'une cellule n'est pas une molécule et s'en distingue au moins par une taille considérablement plus élevée et par le fait qu'elle n'est pas soluble dans les milieux physiologiques. Ainsi, jusqu'à ce jour, la technique sandwich n'a jamais été appliquée à des cellules entières.

Par ailleurs, l'immunocapture de cellules sur un support solide est décrite dans la demande de brevet WO 86/02091 dans laquelle le but est d'éliminer des cellules indésirables dans des échantillons de moelle osseuse destinée à des transplantations. Dans cette demande de brevet, la capture des cellules est réalisée sur des microbilles flottantes et nécessite que l'anticorps utilisé soit relié au support solide par une structure macromoléculaire complexe, appelée réseau-relais, capable d'assurer une orientation privilégiée de l'anticorps par rapport à celle de l'antigène cellulaire correspondant. Aucune application de la technique au dosage quantitatif d'un antigène n'est indiquée dans cette demande.

L'immunocapture de cellules est également décrite dans la demande de brevet WO 84/03151 pour une application analytique. Dans cette demande, le but est d'identifier les groupes tissulaires auxquels appartiennent les cellules examinées (opération généralement dénommée typage HLA). La capture des cellules est effectuée grâce à des anticorps disposés selon une géométrie particulière sur des supports très spécialisés (lamelles de microscope). Les résultats sont obtenus par simple observation visuelle du support et conduisent à des réponses en "tout ou rien". Ainsi, les sytèmes d'immunocapture de cellules décrits jusqu'à ce jour ne conduisent pas à des applications analytiques permettant la détermination quantitative d'un antigène exprimé à la membrane de certaines cellules. De plus, tous ces systèmes peuvent manquer de spécificité car ils reposent sur la reconnaissance des cellules par un unique anticorps.

La méthode de dosage qui fait l'objet de la présente invention, possède des avantages considérables par rapport à toutes les techniques antérieurement connues et utilisées car elle permet de mesurer de façon quantitative plusieurs antigènes caractéristiques chacun d'une sous-population cellulaire, ledites sous-populations étant constitutives d'une population, en un seul temps d'analyse. Ce dosage est réalisé sur des cellules qui n'ont subi aucune intervention chimique ou physique et qui sont dans leur état d'intégrité physiologique. De plus, la méthode de dosage selon l'invention possède les caractéristiques de très haute spécificité, propres aux systèmes à double reconnaissance immunologique mettant en oeuvre 2 anticorps différents spécifiques de 2 antigènes différents portés par la même cellule. Cette méthode est simple, rapide et reproductible. Elle est tout à fait adaptée à l'analyse d'un grand nombre d'échantillons ce qui permet son utilisation à des fins de diagnostic dans les laboratoires de biologie clinique à grand débit.

Ainsi, la présente invention se rapporte à une trousse pour le dosage immunométrique de plusieurs antigènes de surface caractéristiques chacun d'une sous-population cellulaire, lesdites sous-population étant constitutives d'une population, ladite trousse comprenant comme composants :
a) un support solide sur lequel sont fixés par liaison convalente ou par adsorption physique un ou plusieurs anticorps monoclonaux dirigés contre des antigènes de surface communs à toute ladite population cellulaire, autres que les antigènes caractéristiques desdites sous-populations.
b) plusieurs solutions contenant chacune un anticorps monoclonal spécifique chacun dudit antigène caractéristique d'une sous-population cellulaire à doser, marqué par une sonde radioactive ou une sonde enzymatique;
c) dans le cas d'anticorps marqués par une sonde enzymatique, une ou plusieurs solutions apportant les réactifs nécessaires pour révéler l'activité de l'enzyme;
d) éventuellement, un composant additionnel constitué par une solution tampon de lavage et/ou des échantillons permettant l'étalonnage et le contrôle de qualité du dosage.

Le terme cellule, utilisé dans la présente description et dans les revendications qui vont suivre, inclut les cellules humaines, les cellules animales, les cellules de protozoaires et les cellules des micro-organismes (bactéries ou champignons). Concernant les cellules sanguines, la présente invention inclut Les éléments figurés nucléés, tels que les leucocytes et les éléments figurés anucléés, tels que les hématies ou les plaquettes.

La méthode de dosage selon l'invention s'applique à des cellules entières, c'est-à-dire non lysées.

Ces cellules n'ont subi aucune intervention physique ou chimique et elles sont mises en oeuvre dans un état d'intégrité physiologique complète. Cette situation constitue la meilleure garantie d'intégrité des marqueurs membranaires choisis comme cibles de dosage.

Comme support solide, on peut utiliser tout dispositif adapté à la manipulation de suspensions cellulaires et préférentiellement des tubes, des supports magnétiques particulaires ou des plaques rigides ou souples de microtitration en polyéthylène, polystyrène, chlorure de polyvinyle ou nitrocellulose comportant des micropuits. Les anticorps monoclonaux destinés à l'immobilisation des cellules peuvent être fixés sur les supports solides soit par liaison chimique covalente, soit par adsorption physique selon les techniques classique bien connues de l'homme de l'art, telles que celles décrites par STOCKER et HEUSSER J. Immunol. Methods, 1979, vol. 26, p. 87-95. Avantageusement, le support peut être au préalable saturé à l'aide d'une protéine.

Selon l'invention, le ou les anticorps monoclonaux fixés sur le support solide doivent permettre l'immunocapture de la population cellulaire parmi laquelle se trouve la ou les populations cellulaires porteuses des antigènes à doser. Lorsque cette population est constituée de cellules humaines, les anticorps monoclonaux préférés pour l'immunocapture sont les anticorps anti-HLA de classe I qui sont spécifiques de la partie commune des antigènes HLA A, B et C présents sur les leucocytes et de nombreuses autres lignées cellulaires de l'organisme. Parmi ces anticorps, celui dénommé S-classe I, commercialisé par BIOSYS, est particulièrement préféré.

Dans d'autres cas où les cellules examinées sont des cellules humaines et dans tous les cas lorsque ces cellules ne sont pas humaines, des anticorps monoclonaux appropriés au type de cellules examinées peuvent également être utilisés pour l'immunocapture selon l'invention.

L'expression "un anticorps monoclonal marqué par une sonde radioisotopique" signifie que l'anticorps monoclonal porte, soit sur un élément propre de sa structure, par exemple les résidus de tyrosine constitutifs, soit sur un radical approprié qui lui a été fixé, un isotope radioactif permettant de le doser par comptage de la radioactivité qui lui est associée.

L'expression "un anticorps monoclonal marqué par une sonde enzymatique" signifie que l'anticorps monoclonal est couplé à une enzyme qui, associée à l'emploi de réactifs appropriés, permet une mesure quantitative de cet anticorps monoclonal.

Le substrat et les réactifs sont choisis de sorte que le produit final de la réaction ou de la séquence de réaction provoquée par l'enzyme et mettant en oeuvre ces substances soit :
- ou bien une substance colorée ou fluorescente qui diffuse dans le milieu liquide environnant les cellules et qui fait l'objet de la mesure finale spectrophotométrique ou fluorimétrique, respectivement,
- ou bien une substance colorée insoluble qui se dépose sur les cellules et les parois sur lesquelles elles sont fixées et qui peut faire l'objet, soit d'une mesure photométrique par réflexion, soit d'une évaluation à l'oeil éventuellement par rapport à une gamme de teintes étalonnées.

La trousse de dosage peut contenir comme composant additionnel une solution tampon destinée au lavage du support solide après immobilisation et marquage des cellules avec le ou les anticorps portant la sonde choisie.

La trousse de dosage peut aussi contenir comme composants additionnels les échantillons nécessaires à l'étalonnage du dosage ainsi qu'au contrôle de qualité du dosage.

La présente invention a également pour objet un procédé pour le dosage immunométrique de plusieurs antigènes de surface caractéristiques chacun d'une sous-population cellulaire, lesdites sous-populations étant constitutives d'une population, caractérisé en ce qu'il comprend :
- une étape unique pour l'immobilisation spécifique ou immunocapture de ladite population cellulaire sur le support solide en utilisant un ou plusieurs anticorps monoclonaux, préalablement fixé par liaison covalente ou par adsorption physique sur ledit support et capable de reconnaître un antigène présent à la surface des cellules autre que l'antigène à doser et, simultanément, le marquage direct de l'antigène de surface à doser, appartenant à la population cellulaire immobilisée, ou à une de ses sous-populations, par un anticorps monoclonal spécifique de cet antigène à doser, ledit anticorps monoclonal portant une sonde radio-isotopique ou, alternativement, enzymatique ;
- une période d'incubation pour permettre l'immunocapture et le marquage simultanés ;
- le lavage du support solide pour éliminer les cellules indésirables non immobilisées et l'excès d'anticorps monoclonal portant la sonde radio-isotopique ou enzymatique ;
- le dosage proprement dit de chaque antigène à doser dans les sous-populations cellulaires marquées, par comptage de la radioactivité fixée ou, alternativement, après traitement du milieu par le substrat de l'enzyme et éventuellement un ou des réactifs auxiliaires appropriés, par mesure photométrique en transmission ou en réflexion, ou par mesure de l'émission de fluorescence.

La trousse de dosage et le procédé immunométrique selon l'invention s'appliquent de façon préférentielle au dosage des antigènes de surface des éléments figurés du sang humain notamment les leucocytes, plus particulièrement les lymphocytes, les lymphocytes T, les lymphocytes T4, les lymphocytes T8, les lymphocytes B, ainsi que les granulocytes, les monocytes et les plaquettes sanguines.

Une autre application préférentielle est le dosage des antigènes de surface des micro-organismes pathogènes, par exemple le Candida albicans.

En outre, la trousse de dosage et le procédé immunométrique selon l'invention sont particulièrement utiles pour le dosage des antigènes de surface des cellules tumorales, notamment celles des cancers de l'appareil urinaire et celles des hémopathies malignes.

La trousse de dosage et le procédé immunométrique selon l'invention permettent de mesurer des signaux (radioactivité ou lumière absorbée ou émise) dépendant à la fois du nombre de cellules présentes dans la population cellulaire examinée et de la densité de l'antigène mesuré à la surface de ces cellules. La mesure de ces signaux permet une évaluation quantitative du nombre total de molécules de cet antigène qui sont portées par la population ou sous-population cellulaire examinée, que cet antigène soit à rôle structural ou fonctionnel.

Par exemple, dans le cas des marqueurs leucocytaires particulièrement importants en hématologie, on sait que chez les sujets sains, dans la majorité des situations, la valeur moyenne de la densité antigénique ne varie pas de façon importante d'un échantillon à un autre pour une même population cellulaire. Il existe alors une bonne corrélation entre le dénombrement cytologique des cellules portant l'antigène considéré et le signal mesuré selon l'invention qui est proportionnel au nombre total de molécules d'antigène présentes dans l'échantillon examiné. Au contraire, dans certains états pathologiques, la densité des antigènes de surface peut varier pour une même population cellulaire sans que le nombre ou la proportion des cellules positives varie notablement. De tels états pathologiques sont plus efficacement mis en évidence par application du procédé immunométrique selon l'invention ou en utilisant la trousse de la présente invention, qu'ils ne le seraient par une procédure conventionnelle de dénombrement cytologique.

Une autre application de l'invention apparaît en choisissant comme support solide une plaque de microtitration. La trousse de dosage et le procédé immunométrique selon l'invention peuvent alors avantageusement être utilisés pour le dosage sur une seule plaque d'une série d'antigènes de surface caractéristiques de diverses sous-populations constitutives de la population cellulaire examinée. Pour cette application, on peut utiliser, d'une part, des plaques de microtitration prêtes à l'emploi sur lesquelles ont été fixés à l'avance un ou plusieurs anticorps monoclonaux capables de retenir toutes les cellules de la population examinée et disposer, d'autre part, d'une série d'anticorps monoclonaux couplés à un marqueur approprié et spécifiques chacun d'un antigène caractéristique de l'une des sous-populations à évaluer. Ainsi, en une seule manipulation et sur un même support, on peut obtenir le dosage quantitatif de tous les antigènes nécessaires à la caractérisation des sous-populations choisies.

Cette application de la présente invention est illustrée par la caractérisation de l'équipement antigénique de cellules présentant un intérêt en biologie clinique. Un premier cas est représenté par la détermination des groupes tissulaires caractérisant un individu donné, connue sous l'appellation usuelle de typage HLA.

Un deuxième cas est représenté par le typage de cellules tumorales, en particulier pour les malades atteints d'hémopathies malignes, telles que les leucémies ou lymphomes. Cet examen de diagnostic, pratiqué systématiquement, consiste à caractériser le type et l'origine des cellules tumorales du patient par la présence ou l'absence sur ces cellules d'une série d'antigènes de surface convenablement choisis.

En utilisant la trousse selon l'invention, comportant une plaque de microtitration sur laquelle ont été fixés à l'avance un ou plusieurs anticorps monoclonaux capables de fixer toutes les cellules de la population examinée et des solutions de différents anticorps monoclonaux marqués par une sonde enzymatique ou radio-isotopique et spécifiques chacun d'un antigène présent sur les cellules tumorales, on peut identifier et évaluer quantitativement les antigènes caractéristiques de la population des cellules tumorales du patient et les rattacher ainsi à l'un des grands groupes de cancers et notamment d'hémopathies malignes, cliniquement caractérisés. L'application du procédé selon l'invention permet ainsi d'effectuer rapidement, sur un support unique, l'examen qualitatif et quantitatif de l'équipement antigénique des cellules tumorales.

A titre d'illustration d'une autre application de l'invention, on peut citer le cas des lymphocytes T humains, pour lesquels il existe notamment 2 sous-populations de cellules : les lymphocytes caractérisés par la présence du marqueur CD4 et qu'on appellera les lymphocytes T4 positifs ou plus simplement lymphocytes T4 et les lymphocytes caractérisés par la présence du marqueur CD8, qu'on appellera lymphocytes T8 positifs (ou lymphocytes T8).

La mesure du rapport numérique T4/T8 présente un grand intérêt diagnostique en biologie clinique. En effet, il est connu que les modifications du rapport T4/T8 apparaissent dans diverses affections du système immunitaire, telles que les maladies dysimmunitaires, les maladies infectieuses chroniques, les infections virales et notamment les affections à HIV (virus du SIDA)

La trousse de dosage et le procédé immunométrique selon l'invention peuvent être utilisés pour le dosage d'antigènes globalement caractéristiques de la population de lymphocytes T et/ou d'antigènes caractéristiques des sous-populations de lymphocytes T4 et T8. Dans ce cas, on effectue l'immobilisation spécifique des lymphocytes T de l'échantillon examiné sur un support solide et, simultanément, on effectue le marquage direct des antigènes de surface des lymphocytes T4 par un anticorps monoclonal anti-CD4 portant une sonde radio-isotopique ou enzymatique ; de la même manière, on effectue le marquage direct des antigènes de surface des lymphocytes T8 par un anticorps monoclonal anti-CD8 portant une sonde appropriée.

Pour le dosage des lymphocytes T totaux, on utilise de préférence un anticorps monoclonal anti-CD7 (également dénommé anti-T2) portant une sonde appropriée.

Pour obtenir l'immobilisation spécifique des lymphocytes T de l'échantillon, on utilise de préférence un ou plusieurs anticorps monoclonaux capables seuls ou en combinaison de reconnaïtre la totalité des cellules T de l'échantillon, ce qui est le cas des anticorps anti-leucocytes communs (ou anti-CD45) ou des anticorps reconnaissant l'ensemble de la population T (dits "pan-T") tels que les anticorps anti-CD2 (ou anti-T11), anti-CD5 (ou anti-T1), anti-CD7 (ou anti-T2) ou d'autres anticorps pan-T non encore affectés à une classe de différenciation selon les critères OMS.

On peut avantageusement utiliser la méthode immunométrique de l'invention pour le dosage d'antigènes caractéristiques de la population des lymphocytes T et des lymphocytes T4 et T8 sur plusieurs parties du même support solide. La mesure des signaux par comptage de radioactivité, mesure photométrique en transmission ou en réflexion, ou mesure de fluorescence permet de calculer aisément et directement le rapport numérique CD4/CD8.

De la même manière, on peut doser sur un même support solide, les sous-populations appelées lymphocytes T et lymphocytes B, constitutives de l'ensemble de la lignée lymphocytaire.

On peut par exemple, fixer par adsorption un anticorps monoclonal ou un mélange d'anticorps monoclonaux spécifiques de la totalité des antigènes de surface des cellules T sur les parois des micropuits. Ces anticorps monoclonaux permettront d'immobiliser ultérieurement dans les micropuits toute la population des cellules T de l'échantillon examiné. Les plaques ainsi préparées peuvent être lyophilisées et stockées de préférence à 4°C. Cette étape peut être réalisée industriellement et l'on pourra ainsi disposer de plaques prêtes à l'emploi pour les trousses de dosage applicables soit aux lymphocytes T totaux soit à toute sous-population des lymphocytes T.

Les échantillons contenant les cellules à doser et provenant du sang ou de tout liquide biologique approprié, normal ou pathologique, peuvent être utilisés tels quels ou après préparation, notamment par centrifugation en gradient de densité selon les méthodes déjà connues et en particulier en milieu à forte densité comme par exemple, le FICOLL-PAQUE®, commercialisé par Pharmacia. Pour le dosage des lymphocytes sanguins, on peut également traiter l'échantillon de sang à doser avec une solution appelée tampon de lyse qui lyse les globules rouges.

Des parties aliquotes de la suspension cellulaire appropriée sont placées au contact du support solide, par exemple dans les micropuits d'une plaque de microtitration préalablement préparée, en même temps que la solution faisant partie de la trousse de dosage et contenant l'anticorps monoclonal spécifique de la population cellulaire viséeet portant une sonde appropriée, c'est-à-dire un traceur radio-isotopique ou enzymatique. Ainsi une sonde radio-isotopique peut être réalisée, par exemple par marquage de l'anticorps monoclonal à l'iode 125 ou l'iode 131, par exemple en présence de chloramine T selon un procédé connu (F.C. GREENWOOD, W.M. HUNTER et Coll, Biochem. J., 1963, 89, 114) ; ou bien une sonde enzymatique peut être réalisée en conjuguant l'anticorps monoclonal à une enzyme telle que la phosphatase alcaline, la peroxydase, la β-galactosidase ou l'acétylcholinestérase, selon les méthodes connues (voir par exemple M. O'SULLIVAN Methods in Enzymology 1981, 73, 147) ou selon une méthode qui en découle. Dans certains cas, pour éviter certains inconvénients liés à la manipulation de substances radioactives et à la durée limitée de validité des réactifs, les enzymes seront utilisées de préférence aux sondes radio-isotopiques.

La période d'incubation, c'est-à-dire la durée nécessaire pour l'immobilisation et le marquage simultanés des cellules est de préférence inférieure ou égale à 1 heure. Pendant ce délai, le support solide peut éventuellement être centrifugé pour améliorer l'immobilisation des cellules. Le support solide, la plaque de microtitration par exemple, est ensuite lavé pour éliminer en même temps les cellules non fixées et l'anticorps monoclonal portant une sonde enzymatique ou radio-isotopique, en excès.

Lorsque l'on utilise une sonde radio-isotopique, comme par exemple l'iode 125, la radioactivité associée aux cellules est comptée dans un compteur gamma selon toute modalité appropriée et par exemple après solubilisation des cellules par une solution alcaline (par exemple une solution de soude) et récupération de la solution contenant la radioactivité à l'aide d'un tampon absorbant.

Lorsque l'on utilise une sonde enzymatique sur l'anticorps monoclonal, l'apparition d'un produit coloré ou fluorescent est obtenue en ajoutant au support solide sur lequel a été fixée la population cellulaire portant l'antigène à doser une solution contenant le substrat de l'enzyme et un ou plusieurs réactifs auxiliaires permettant d'obtenir finalement comme produit de réaction, soit un produit coloré soluble dans le milieu, soit un produit coloré insoluble, soit un produit fluorescent soluble, comme cela a été expliqué précédemment. On mesure ensuite le signal lumineux provenant des échantillons ainsi traités, à l'aide de l'appareillage adapté à chaque cas : photomètre en transmission ou en réflexion ou fluorimètre respectivement. Lorsque le support solide est une plaque de microtitration, la lecture du signal lumineux peut être effectuée séquentiellement dans tous les puits d'une même plaque par l'emploi de lecteurs automatisés d'usage courant dans les laboratoires de biologie, tels que par exemple le lecteur de plaque Titertek® ou le lecteur de plaque Fluoroscan®, pour les lectures spectrophotométriques ou fluorométriques respectivement.

En utilisant comme sonde enzymatique la phosphatase alcaline, le couplage de cette enzyme avec l'anticorps monoclonal est effectué selon la méthode proposée par Boehringer Mannheim - Biochemica. Les substrats préférentiels de cette enzyme sont le paranitrophénylphosphate pour une lecture finale spectrophotométrique ou le méthyl-4- umbelliféryl phosphate pour une lecture fluorométrique ou le bromo-5 chloro-4 indolyl-3 phosphate pour obtenir un produit de réaction coloré insoluble. On peut de même utiliser comme sonde enzymatique la β-galactosidase dont les substrats préférentiels seront alors l'orthonitrophényl β-D-galactopyranoside ou le méthyl-4 umbelliféryl β-D-galactopyranoside.

Préférentiellement, on peut coupler les anticorps monoclonaux à la peroxydase. Dans ce cas, le procédé de couplage est dérivé de celui décrit par M.B. WILSON et P.K. NAKANE in Immunofluorescence and Related Staining Techniques. W. Knapp, K. Kolubar, G. Wicks ed , Elsevier/North Holland. Amsterdam, 1978, p. 215-224. Les modifications introduites par rapport au protocole initial de préparation du conjugé enzymatique portent sur les points suivants :
- rapport molaire peroxydase/anticorps égal à 3, contre 2 dans le protocole,
- oxydation moins poussée des motifs glucidiques de la peroxydase par diminution de 33 % de la concentration proposée en periodate de sodium.

Les réactifs utilisés pour révéler la peroxydase conjuguée aux anticorps monoclonaux contiennent de l'eau oxygénée, substrat de l'enzyme, et un chromogène approprié par exemple de l'orthophénylènediamine ou l'acide azino-2,2′ bis (éthyl-3 benzo-thiazoline sulfonique-6) ou ABTS pour obtenir un produit final de réaction coloré et soluble dans le milieu ou bien la diamino-3,3′ benzidine ou l'amino-3 éthyl-9 carbazole ou le chloro-4 α-naphtol pour obtenir un produit final de réaction insoluble, ou bien l'acide parahydroxyphényl propionique pour obtenir un produit de réaction fluorescent soluble dans le milieu.

Selon une forme préférentielle, la trousse selon l'invention pour le dosage d'antigènes caractéristiques des lymphocytes T, T4 et T8 comprend :
a) une plaque de microtitration dans les puits de laquelle ont été fixés un ou plusieurs anticorps monoclonaux antilymphocytes T.
b1) une solution contenant au moins un anticorps monoclonal antilymphocytes T marqué à la peroxydase.
b2) une solution contenant au moins un anticorps monoclonal anti-antigène CD4 marqué à la peroxydase.
b3) une solution contenant au moins un anticorps monoclonal anti-antigène CD8 marqué à la peroxydase.
c1) une solution contenant de l'eau oxygénée, substrat de l'enzyme, dans un tampon approprié.
c2) une solution contenant le chromogène utilisé pour révéler l'expression de l'activité de l'enzyme.

Un autre mode préférentiel de réalisation de l'invention est l'utilisation d'anticorps monoclonaux couplés à l'acétylcholinestérase.

L'acétylcholinestérase est couplée à l'anticorps en utilisant préférentiellement un procédé dérivé de celui décrit dans le brevet français n^{o} 2 550 799 ou un procédé qui comporte schématiquement la préparation de fragments de l'anticorps par une technique connue, la modification de l'enzyme par réaction avec un agent hétérobifonctionnel approprié et enfin le couplage des produits ainsi obtenus. D'autres procédés connus de construction de conjugués immunoenzymatiques peuvent aussi être utilisés dans ce cas.

La révélation de l'activité enzymatique spécifiquement liée à l'antigène cellulaire reconnu par le conjugé à l'acétylcholinestérase est réalisée de préférence selon la technique bien connue qui emploie l'acétylthiocholine comme substrat de l'enzyme et le réactif d'Ellman, ou acide dithio-5,5′ nitro-2 benzoïque comme chromogène, selon toute variante adaptée au cas examiné, par exemple celle décrite par Pradelles et al. Anal. Chem. 57 (1985) 1170-1173.

Les chromogènes cités sont utilisés tels quels ou sous forme de sels solubles dans l'eau.

Les résultats du dosage des antigènes selon l'invention peuvent être exprimés selon toute modalité appropriée à l'examen effectué. Plus particulièrement on peut exprimer ces résultats soit en nombre total de molécules d'un antigène particulier (par exemple l'antigène CD4) présentes dans un volume donné de l'échantillon examiné (par exemple par microlitre de sang), soit par le rapport du nombre des molécules d'un antigène au nombre des molécules d'un autre antigène dans l'échantillon examiné (par exemple le rapport des antigènes CD4 aux antigènes CD8 - ou rapport CD4/CD8 dans l'échantillon de sang examiné).

Pour déterminer le nombre de molécules d'un antigène particulier, dans l'échantillon examiné, on utilisera de préférence une gamme d'étalonnage constituée de cellules ou de préparations cellulaires appropriées, portant l'antigène à doser et qui auront été préalablement calibrées par une méthode de référence connue. Ces étalons seront de préférence constitués soit par des cellules identiques dans leur provenance aux cellules qui feront l'objet du dosage, soit par des cellules de lignées cellulaires établies portant l'antigène recherché, soit par des préparations, par exemple de membranes, provenant de ces cellules.

Ces étalons sont alors traités exactement comme les échantillons à examiner. Les signaux qui en découlent servent à construire une gamme d'étalonnage à laquelle sont rapportés les signaux mesurés avec les échantillons à examiner. Les calculs ultérieurs sont classiques.

Pour déterminer le rapport des nombres de molécules de deux antigènes dans l'échantillon à examiner, on peut utiliser le système d'étalonnage décrit ci-dessus et calculer finalement le rapport recherché. Plus simplement dans de nombreux cas, on peut calculer directement le rapport des signaux spécifiques obtenus avec chacun des antigènes recherchés, le corriger le cas échéant par des facteurs connus tels que le rapport des dimensions des prises d'essai mises en oeuvre et on obtient directement le rapport recherché.

La méthode de dosage immunométrique selon l'invention est simple, rapide et reproductible. Son utilisation est tout à fait adaptée à l'analyse d'un grand nombre d'échantillons. Pour en comprendre les avantages par comparaison avec les autres méthodes décrites, il convient d'en analyser les différentes étapes.

L'immobilisation des cellules sur le support solide est la phase du dosage qui présente habituellement le plus de difficultés ou qui est la plus critique dans sa réalisation. Le moyen souvent utilisé est la fixation chimique des cellules par le glutaraldéhyde ou le méthanol dans des cupules traitées ou non à la poly-L-lysine (VAN LEUVEN F. et Coll., J. Immunol. Methods, 1978, 23, 109). Toutefois, les fixations chimiques ainsi mises en oeuvre peuvent diminuer ou même supprimer la détection spécifique recherchée ou au contraire induire des marquages faussement positifs de cellules ce qui est un inconvénient très grave (DROVER et MARSHALL J. Immunol. Methods, 1986, 90, 275-281).

De plus la réalisation du procédé par fixation chimique nécessite plusieurs étapes : la centrifugation des cellules, la préparation du mélange de fixateur, la fixation puis les lavages des cellules fixées.

La dessiccation des cellules à 37°C, suivie ou non d'une fixation au méthanol dans les micropuits a aussi été proposée (BAUMGARTEN, J. Immunol. Methods, 1986, 94, 91-98). En fait, la déshydratation des cellules à 37°C, outre qu'elle peut altérer certains antigènes fragiles, perméabilise la membrane plasmique péricellulaire ce qui facilite l'immunomarquage d'antigènes intracytoplasmiques en plus du marquage des antigènes de surface, conduisant ainsi à des bruits de fond gênants ou à des résultats faussement positifs lorsque l'on souhaite une mesure limitée aux antigènes de surface.

De plus, la reproductibilité de ce procédé est incertaine ; en effet, la décantation des cellules dans les micropuits du dosage et la dessiccation des cellules peuvent varier d'une expérience à l'autre. Enfin, ce dosage est long à réaliser car l'étape de dessiccation des cellules nécessite à elle seule un délai supérieur à 2 heures.

L'immobilisation de populations lymphocytaires a été aussi obtenue grâce à l'emploi d'anticorps polyclonaux adsorbés dans des micropuits (STOCKER et HEUSSER J. Immunol. Methods, 1979, 26, 87-95). Outre de permettre l'immobilisation de cellules étrangères à la seule population que l'on souhaite analyser, les anticorps polyclonaux présentent aussi l'inconvénient de réagir avec les antigènes destinés à être mesurés par l'anticorps marqué, ce qui réduit d'autant le signal finalement mesuré.

L'utilisation d'anticorps monoclonaux ayant une haute spécificité et affinité adsorbés ou fixés sur le support solide et notamment dans les puits du dosage permet la capture exclusive des cellules recherchées, les autres populations cellulaires non retenues étant éliminées au cours des lavages effectués en fin de marquage des antigènes à doser. De plus, aucun agent chimique ou physique ne modifie les caractéristiques des antigènes à cette étape car les différentes opérations destinées à fixer chimiquement ou physiquement les cellules au support sont supprimées.

Ainsi, selon la présente invention, il a été trouvé que l'immobilisation des cellules par des anticorps monoclonaux est un procédé qui permet de simplifier l'étape d'immobilisation des cellules portant l'antigène à doser, tout en rendant les résultats plus fiables.

Les dosages immunométriques appliqués à des populations cellulaires sont réalisés généralement par marquage des cellules selon une méthode indirecte en 2 ou 3 étapes successives, la sonde qui apporte le signal spécifique étant fixée sur les cellules au cours de la dernière étape du procédé de marquage. Dans le marquage des cellules en deux étapes, les principaux réactifs mis en jeu utilisent des anticorps anti-immunoglobulines (anti-Ig) couplés à la β-galactosidase (COBBOLD et Coll. J. Immunol. Methods, 1971, 44, 125-133) ou à la phosphatase alcaline (HESSIAN J. Immunol. Methods, 1986, 91, 29-34) ou marqués à l'iode 125 (SAVION J. Immunol. Methods 1987, 97, 49-56). La technique de marquage au réactif peroxydase anti-peroxydase est, elle, réalisée en 3 étapes (VAN LEUVEN 1978).

Un autre procédé, lui aussi en 3 étapes, fait intervenir un anticorps monoclonal spécifique du marqueur antigénique à analyser, puis des anticorps anti-Ig de souris porteurs de biotine et enfin un conjugué streptavidine-peroxydase (BAUMGARTEN, 1986) ou streptavidine-phosphatase alcaline (IGIETSEME et Coll. J. Immunol. Methods, 1987, 97, 123-131).

Le procédé selon l'invention comportant l'utilisation simultanée, en une seule étape, d'un processus d'immunocapture des cellules entières, sans intervention physique ou chimique sur les cellules, et le marquage de tout ou partie de ces cellules par un ou plusieurs anticorps monoclonaux portant directement une sonde radio-isotopique ou enzymatique, permet, pour la première fois, le dosage quantitatif sur les cellules elles-mêmes d'antigènes membranaires choisis.

Selon l'invention, le marquage direct des cellules immobilisées immunologiquement permet :
- la simplification de la méthode de dosage par suppression des manipulations intermédiaires répétées entre les étapes successives du marquage dans le cas du marquage indirect : centrifugation des cellules, élimination des réactifs de marquage, remise en suspension ;
- une économie de réactifs ;
- une fiabilité améliorée par diminution du nombre d'étapes et de manipulations ;
- un gain de temps ;
- la possibilité de traiter en même temps, avec l'emploi exclusif de matériels et d'appareillages usuels, de grands nombres d'échantillons.

La période d'incubation pour l'immobilisation de la population cellulaire et simultanément le marquage direct des antigènes de la sous-population cellulaire à doser est courte. Elle est inférieure ou égale à 1 heure dans le cas du dosage des lymphocytes T et des sous-populations de lymphocytes T4 et T8.

Après le lavage du support solide, le dosage proprement dit est effectué par l'observation d'un signal précis et simple à mesurer avec des appareillages usuels : radioactivité, absorption ou émission lumineuse.

Ainsi l'ensemble du procédé selon l'invention présente de nombreux avantages : il est rapide, fiable, économique et simple.

Le procédé selon l'invention permet de doser plusieurs antigènes de surface caractéristique chacun d'une sous-population cellulaire dans une large gamme de concentrations cellulaires.

La sensibilité de la méthode vis-à-vis du nombre de cellules dépend de la densité antigénique de la population cellulaire dosée. Pour chaque antigène, on peut, si on le souhaite, définir la concentration minimale molaire en antigène pouvant être mesurée par le procédé selon l'invention.

Ainsi, par exemple, lorsque le support solide choisi est une plaque de microtitration et que les cellules examinées sont des lymphocytes humains, on a observé que l'on obtient des mesures significatives lorsque le nombre de cellules analysées est compris entre quelques centaines et environ 200 000 par micropuits de 200 µl, la limite inférieure étant imposée par la densité de l'antigène mesuré sur les cellules examinées et la sensibilité de la technique de détection choisie, alors que la limite supérieure dépend essentiellement de la dimension et de la géométrie du support solide. Il en est de même lorsque le support solide est constitué de tubes.

On a vérifié que les signaux enregistrés (comptage de radioactivité ou mesures photométriques) permettent d'obtenir des courbes d'étalonnage régulières et satisfaisantes en fonction du nombre de cellules mises en oeuvre, dans les conditions usuelles de manipulation.

Par ailleurs, on peut améliorer la sensibilité de la méthode si nécessaire en fixant simultanément sur un même antigène de surface plusieurs anticorps monoclonaux différents, spécifiques de plusieurs épitopes différents du même antigène. Ceci a été vérifié par un dosage des antigènes CD4 sur des cellules de la lignée Ichikawa (lignée T humaine), pour lequel on a utilisé simultanément les anticorps OKT4 et ST4 marqués à l'iode 125. Le signal mesuré a augmenté d'environ 50 % par rapport aux signaux obtenus avec chacun des anticorps anti-CD4 utilisé seul.

Dans les exemples qui vont suivre, on utilisera indifféremment les termes suivants ou leurs abréviations :
- BSA: : albumine de sérum bovin, ou sérum albumine bovine
- PBS: : tampon phosphate salin à pH 7,4
- POD: : peroxydase
- Ig G: : immunoglobuline G
- Ig M: : immunoglobuline M
- Ig G anti-T ou anti-T: : anticorps antilymphocytes T
- Ig G anti-CD4 ou anti-CD4: : anticorps anti-antigène CD4
- Ig G anti-CD8 ou anti-CD8: : anticorps anti-antigène CD8
- cpm: : coups par minute
- dpm: : désintégrations par minute

### Exemple 1

Dosage immunoenzymométrique de la concentration moléculaire des antigènes CD4 et CD8 à partir d'échantillons de sang humain comprenant un marquage des anticorps à la peroxydase.

### A) Préparation de la trousse de dosage

### a) Préparation de la plaque

On utilise une plaque de microtitration en plastique commercialisée par NUNC (Référence 64394) comportant 96 micropuits. On place par micropuits 200 µl d'une solution contenant l'anticorps monoclonal purifié anti-CD2 (dénommé ST 11) utilisé pour immobiliser les lymphocytes T totaux, c'est-à-dire pour effectuer leur immunocapture. Cet anticorps commercialisé par BIOSYS, Compiègne, France, sous la référence ST 11 est utilisé à la concentration de 10 µg/ml dans un tampon phosphate salin à pH 7,4 (PBS).

L'adsorption de l'anticorps monoclonal s'effectue à 4°C pendant 12 heures. On élimine l'anticorps en excès par retournement de la plaque.

On prépare une solution contenant 0,1 % de gélatine et 0,5 % de BSA dans un tampon phosphate salin. On introduit 250 µl de cette solution par micropuits, afin de saturer la surface des puits en protéine, ce qui est obtenu après 1 heure à 37°C ; on lave les plaques 3 fois à l'aide de tampon phosphate salin. Les plaques ainsi préparées sont lyophilisées et stockées à 4°C dans un sachet plastique scellé.

### b) Préparation de la solution de conjugué anticorps monoclonal peroxydase

On utilise la peroxydase (POD) commercialisée par Boehringer Mannheim Biochemica (référence 814 393).

Le procédé de couplage entre l'anticorps et la peroxydase est celui décrit par M.B. WILSON et P.K. NAKANE in Immunofluorescence and Related Techniques (W. KNAPP, K. KOLUBAR, G. WICKS ed., 1978, Elsevier/North Holland, Amsterdam - p. 215-224), excepté que l'on met en oeuvre pour l'oxydation de la peroxydase, 1,5 mg de POD dans 0,36 ml d'eau distillée et qu'on ajoute 50 µl d'une solution 0,2 M de periodate de sodium. Le produit ainsi obtenu est couplé à 2 mg d'IgG anti-CD4 contenus dans 500 µl de tampon carbonate. Après traitement par le borohydrure de sodium et dialyse contre le PBS le conjugué IgG-POD est stérilisé par filtration sur membrane 0,22 µm et conservé dans des conditions stériles à la concentration de 0,5 mg d'IgG par ml, à 4°C dans des tubes de verre. Le réactif est stable pendant au moins 1 an.

De la même manière on prépare le conjugué IgG-anti CD8-POD. On peut de la même manière préparer des conjugués IgM-POD.

### c) Préparation du réactif de révélation

Le réactif de révélation est obtenu de la façon suivante : on prépare un tampon citrate 0,1 M en dissolvant de l'acide citrique monohydraté à 2,1 % dans de l'eau et en ajustant à pH 5 par addition de soude 7N. On ajoute alors 30 mg de dichlorhydrate d'orthophénylènediamine à 20 ml du tampon citrate obtenu puis on ajoute, au dernier moment, 40 µl d'eau oxygénée (substrat de l'enzyme) à 30 % par 20 ml de tampon citrate contenant l'orthophénylènediamine.

### B) Procédé immunométrique

### a) Séparation des cellules

On prélève 2 ml de l'échantillon de sang à doser. On les mélange à 2 ml de tampon phosphate salin et on dépose ce mélange par-dessus 3 ml de FICOLL-PAQUE® (commercialisé par Pharmacia). Par centrifugation à 400 x g pendant 30 minutes à température ambiante, il se forme un anneau de suspension contenant les cellules mononucléées. On récupère la totalité de cette suspension sous un volume de 1 ml et l'on distribué 6 x 100 µl de la suspension ainsi obtenue dans 6 micropuits de la plaque préparée préalablement.

### b) Incubation des cellules

On dilue au centième le conjugué POD-anticorps obtenu précédemment à l'aide de PBS contenant 1 % de substance protéinée telle que BSA ou lait écrémé en poudre et l'on dépose 100 µl de cette solution par micropuits, à savoir :
- 2 micropuits sont remplis avec chacun 100 µl de la solution du conjugué POD-anti-CD4.
- 2 micropuits sont remplis avec chacun 100 µl de la solution du conjugué POD-anti-CD8.
- 2 micropuits témoins sont remplis avec chacun 100 µl de solution à 1 % de substance protéinée (blanc de réaction).

Pour améliorer la fixation des cellules sur le support, on centrifuge la plaque pendant 3 minutes à 150 x g après attente de 1 heure à température ambiante.

### c) Révélation de l'enzyme fixée et mesure

On vide les micropuits par retournement de la plaque. On lave les micropuits 4 fois par 200 µl de PBS. On ajoute dans chaque puits 200 µl du réactif de révélation préparé extemporanément. On laisse incuber pendant 20 minutes à température ambiante en lumière atténuée. On mesure la densité optique au spectrophotomètre à 492 nm (appareil Titertek Multiskan® type 310 C - Flow Laboratories).

### d) Etalonnage et expression des résultats

Pour réaliser l'étalonnage de ces essais, une préparation de lymphocytes humains totaux préalablement calibrée en antigènes CD4 et CD8 par la technique cytofluorométrique de PONCELET et al. (J. Immunol. Methods 1985, 85, 65-74) a été utilisée. Des prélèvements aliquotes connus de cette préparation de référence ont servi à constituer les 2 gammes d'étalonnage relatives à l'antigène CD4 et à l'antigène CD8 respectivement, par rapport auxquelles sont calculés les nombres d'antigènes correspondant à chaque échantillon.

A titre indicatif, le tableau 1 ci-après montre pour 20 échantillons de sang humain provenant de donneurs sains, les valeurs de densité optique obtenues, les nombres de molécules d'antigènes CD4 et CD8 correspondants et les rapports de ces nombres d'antigènes qui en découlent (rapport CD4/CD8).

**TABLEAU 1**

| Numéro du donneur | Antigène CD4 | | Antigène CD8 | | Rapport molaire CD4/CD8 |
|---|---|---|---|---|---|
| | Densité optique | Nombre de molécules d'antigène (en millions) par µl de sang | Densité optique | Nombre de molécules d'antigène (en millions) par µl de sang | |
| 1 | 0,64 | 23 | 1,29 | 92 | 0,25 |
| 2 | 0,71 | 27 | 0,75 | 37 | 0,73 |
| 3 | 0,82 | 32 | 1,10 | 81 | 0,39 |
| 4 | 0,49 | 17 | 0,55 | 23 | 0,74 |
| 5 | 0,74 | 29 | 0,89 | 52 | 0,56 |
| 6 | 0,88 | 38 | 0,92 | 55 | 0,69 |
| 7 | 0,74 | 29 | 0,72 | 36 | 0,80 |
| 8 | 0,75 | 29 | 0,69 | 33 | 0,88 |
| 9 | 0,48 | 16 | 0,80 | 41 | 0,39 |
| 10 | 1,00 | 50 | 1,08 | 80 | 0,62 |
| 11 | 0,61 | 22 | 0,69 | 33 | 0,67 |
| 12 | 0,73 | 28 | 0,85 | 46 | 0,61 |
| 13 | 0,51 | 18 | 0,66 | 30 | 0,60 |
| 14 | 0,65 | 24 | 1,06 | 76 | 0,31 |
| 15 | 0,92 | 42 | 0,87 | 48 | 0,87 |
| 16 | 0,77 | 31 | 0,98 | 63 | 0,49 |
| 17 | 0,68 | 25 | 1,01 | 67 | 0,37 |
| 18 | 0,85 | 36 | 0,96 | 61 | 0,59 |
| 19 | 0,91 | 41 | 0,90 | 52 | 0,79 |
| 20 | 1,00 | 50 | 1,02 | 69 | 0,72 |

### Exemple 2

Dosage immunoenzymométrique de la concentration moléculaire des antigènes CD4 et CD8 à partir d'échantillons de sang humain comprenant un marquage des anticorps à la phosphatase alcaline.

Ce dosage est réalisé comme celui de l'exemple 1. La préparation du conjugué anticorps monoclonal - phosphatase alcaline est effectuée selon la méthode indiquée par Boehringer Mannheim - Biochemica (Ref. 567 744). Après une heure d'incubation des cellules à doser avec ce conjugué, on révèle l'enzyme fixée par addition d'une solution de paranitrophénylphosphate à 1 mg/ml dans un tampon diéthanolamine à 1,2 % dans l'eau distillée ajusté à pH 9,8 avec une solution diluée d'acide chlorhydrique. Après 2 heures à 37°C, on mesure la densité optique au spectrophotomètre à 405 nm.

Les résultats sont calculés et exprimés comme dans l'exemple 1.

### Exemple 3

Dosage immunoradiométrique de la concentration moléculaire des antigènes CD4 et CD8 à partir d'échantillons de sang humain comprenant un marquage des anticorps à l'iode 125.

La préparation de l'anticorps marqué à l'iode 125 est effectuée selon la technique décrite par F.C. Greenwood, V.M. Hunter et Coll., Biochem. J., 1963, 89, 114.

On mélange 50 µg d'anticorps monoclonal anti-CD4 ou anti-CD8 contenu dans 50 µl de tampon phosphate salin à pH 7,2 avec 37 MBq d'iode 125 sous forme d'iodure de sodium et 30 µl d'une solution de chloramine T dans un tampon phosphate salin contenant 0,33 mg de chloramine T par ml. Après 1 minute sous agitation, on arrête la réaction de marquage de l'anticorps monoclonal en ajoutant 100 µl d'une solution de métabisulfite de sodium dosée à 2,5 mg/ml. La solution ainsi préparée est passée sur une colonne PD 10 (Pharmacia - Sephadex® G25M) et l'on récupère dans l'effluent la fraction contenant l'anticorps radiomarqué. Le dosage est réalisé en introduisant dans 4 micropuits un volume de 100 µl de suspension cellulaire contenant les cellules mononucléées du sang humain préparées comme dans l'exemple 1. On dépose ensuite 100 µl de solution diluée d'anticorps radioactif dans un tampon PBS contenant 5 % de BSA de façon à introduire 150 000 cpm par puits ; 2 micropuits sont remplis avec chacun 100 µl de la solution du conjugué anti-CD4-125-I, 2 micropuits sont remplis avec chacun 100 µl de la solution du conjugué anti-CD8-125-I. La fixation des cellules est améliorée par centrifugation de la plaque pendant 3 minutes à 150 x g après 1 heure d'incubation à températue ambiante. Les micropuits sont vidés par retournement de la plaque. On lave les micropuits 4 fois par 200 µl de PBS par puits. On introduit ensuite 75 µl de solution d'hydroxyde de sodium 1 M dans chaque puits. Après 10 minutes, le contenu de chaque puits est récupéré avec un tampon absorbant avant comptage de la radioactivité au compteur γ (compteur multipuits LKB).

Les résultats sont calculés et exprimés comme dans l'exemple 1, les valeurs des densités optiques étant remplacées par les résultats de comptage en dpm.

### Exemple 4

Vérification de la validité de la méthode.

Sur 20 prélèvements de sang humain, on a mesuré les antigènes CD4 et CD8 des cellules T humaines en suivant la méthode immunométrique à la peroxydase décrite dans l'exemple 1. Sur les mêmes échantillons de sang, on a déterminé par ailleurs en utilisant la technique conventionnelle (W.W. ERBER et Coll. Lancet, 1984, (8385), 1042-1045) de dénombrement cytologique le rapport du nombre de cellules T4 positives au nombre de cellules T8 positives.

Le coefficient de corrélation entre le rapport CD4/CD8 obtenu par dosage immunoenzymométrique et le rapport T4/T8 obtenu par dénombrement cytologique est r=0,72.

De même, sur 7 autres prélévements de sang humain, on a comparé le rapport CD4/CD8 déterminé par la méthode immunoradiométrique utilisant le marquage à l'iode 125 selon le procédé de la présente invention et le rapport de cellules T4/T8 déterminé par dénombrement des cellules. Le coefficient de corrélation est r=0,87.

Dans les 2 cas, les coefficients de corrélation obtenus démontrent que malgré une concordance d'ensemble satisfaisante entre les résultats des 2 techniques l'information apportée par les 2 rapports n'est pas équivalente, ce qui est évident puisque le dosage des antigènes selon l'invention prend en compte, non seulement le nombre de cellules positives comme c'est le cas pour la méthode cytologique, mais aussi la densité de l'antigène considéré sur les celules positives de chaque échantillon. L'information donnée par la technique décrite dans la présente invention est donc plus complète que celle donnée par la technique conventionnelle de référence (W.W ERBER et Coll. Lancet, 1984, (8385), 1042-1045).

### Exemple 5

Dosage immunoenzymométrique de la concentration moléculaire des antigènes CD4 et CD8 des lymphocytes T à partir d'échantillons de sang humain comprenant un marquage des anticorps à la peroxydase.

Cet exemple est destiné à montrer que l'on peut diminuer la durée nécessaire au dosage, par rapport aux conditions décrites dans l'exemple 1, en modifiant d'une part le procédé de séparation des cellules et d'autre part le temps d'incubation, nécessaire à l'étape d'immunocapture et de marquage des cellules.

### A) Influence du procédé de séparation des cellules du sang total

### a) Technique par centrifugation brève :

On prélève 0,5 ml de l'échantillon de sang à doser que l'on mélange à 1,5 ml de tampon phosphate salin. Dans un tube à hémolyse de 5 ml, on introduit 1,5 ml de FICOLL-PAQUE®, commercialisé par Pharmacia et l'on dépose à la surface de la couche de FICOLL® l'échantillon de sang dilué dans le PBS. Après 5 minutes de centrifugation à 900 x g, à température ambiante, on prélève l'anneau de suspension contenant des cellules mononuclées sous un volume de 0,5 ml. Cet échantillon est additionné de 1,5 ml de PBS.

### b) Technique par lyse des érythrocytes :

Une autre méthode rapide pour la séparation de cellules du sang est l'utilisation d'un tampon qui lyse les globules rouges. Le tampon de lyse, utilisé à titre d'exemple non limitatif, a la composition suivante :
chlorure d'ammonium : 8,29 g
carbonate acide de potassium : 1 g
sel disodique de l'acide éthylène diaminetétracétique 0,0307 g pour 1 litre d'eau distillée, le pH étant ajusté à 7,3.

On mélange 5 ml de tampon de lyse et 0,250 ml de sang. Après 10 minutes sous agitation, on centrifuge à 600 x g pendant 10 minutes. Le culot des cellules formé est repris dans 1 ml de tampon PBS.

En effectuant ensuite le dosage des antigènes CD4 et CD8, dans les conditions de l'exemple 1, et par comparaison au protocole de référence décrit dans l'exemple 1 pour l'isolement des cellules mononucléées du sang, on a vérifié que l'une et l'autre de ces 2 variantes de la méthode de séparation des cellules mononucléées du sang total ont bien permis de recueillir la totalité des lymphocytes des échantillons de sang examinés.

### B) Influence du temps d'incubation pour l'immunocapture et le marquage des cellules.

Afin de vérifier si le délai de 1 heure utilisé à l'exemple 1 ne peut pas être réduit pour accélérer le déroulement du dosage, on a comparé les résultats obtenus pour des échantillons identiques contenant chacun 20 000 cellules mononucléées par puits, lorsque l'on fait varier de 10 minutes à 1 heure le délai accordé à l'étape de capture et marquage des cellules. Les conditions opératoires étant par ailleurs celles de l'exemple 1, les résultats obtenus sont présentés dans le tableau 2.

**TABLEAU 2**

| Temps d'incubation (minutes) | Densité optique | | | |
|---|---|---|---|---|
| | Antigène CD4 | | Antigène CD8 | |
| | Essai | Blanc * | Essai | Blanc * |
| 10 | 0,322 | 0,037 | 0,329 | 0,023 |
| 20 | 0,438 | 0,049 | 0,401 | 0,025 |
| 30 | 0,620 | 0,055 | 0,498 | 0,027 |

| | | | | |
|---|---|---|---|---|
| * Ces valeurs de la densité optique correspondent au blanc de réactifs obtenu en l'absence des cellules. Les valeurs témoins obtenues en présence des cellules et en omettant soit le conjugué soit le substrat ne sont pas supérieures aux valeurs indiquées. | | | | |

Ces résultats montrent que :
- Le signal non spécifique (blanc de réactifs) reste à des valeurs toujours faibles, d'autant plus basses que le temps de présence du conjugué est plus court.
- Le signal spécifique atteint, dès 10 minutes de contact, une valeur exploitable analytiquement avec précision et reproductibilité. Ceci indique qu'il est possible, par rapport aux conditions de l'exemple 1, de réduire significativement la durée d'exécution du dosage sans perte importante sur la précision des résultats.

En pratique et si l'on cumule les gains de temps que l'on peut obtenir au niveau de la préparation de l'échantillon de sang et au niveau de l'immunocapture, il est possible avec la trousse et le procédé de l'invention de doser les antigènes CD4 et CD8 (à titre d'exemples non limitatifs) d'échantillons de sang total, à raison de 10 ou 20 échantillons par plaque de 96 puits, dans un délai total (depuis la réception des échantillons au laboratoire) qui ne dépasse pas 1 heure. Ce niveau de productivité est supérieur à celui de toutes les techniques alternatives connues à ce jour.

### Exemple 6

Dosage de différents antigènes exprimés sur les lymphocytes T humains activés.

L'activation des lymphocytes T est un processus physiologique qui intervient précocement chaque fois que le système immunitaire est sollicité, comme par exemple dans les pathologies infectieuses, dans les transplantations d'organes, dans certaines maladies dysimmunitaires. Ce processus naturel est aussi couramment mis en oeuvre in-vitro au laboratoire dans des tests tels que notamment les réactions lymphocytaires mixtes ou les tests de transformation lymphoblastique. Dans ce dernier cas l'activation polyclonale est obtenue par l'emploi d'agents tels que la phytohémagglutinine (PHA), la concanavaline A ou d'autres lectines. L'activation des lymphocytes T s'accompagne d'un considérable accroissement d'expression de plusieurs marqueurs membranaires et notamment l'antigène CD25 (récepteur de l'interleukine 2) ou l'antigène CD2. Ces antigènes constituent donc d'excellents marqueurs de l'état d'activation et leur dosage présente un grand intérêt à la fois en biologie clinique et pour les travaux de laboratoire comme indiqué ci-dessus, en évitant dans tous les cas l'emploi de réactifs radioactifs.

Ces antigènes sont mesurés en utilisant la méthode décrite à l'exemple 1 avec les spécifications précisées dans le tableau 4.

**TABLEAU 3**

| Mesure des antigènes de surface des lymphocytes T activés | | | | |
|---|---|---|---|---|
| Antigène mesuré | Anticorps utilisé pour l'immunocapture, commercialisé par | | Anticorps marqué à la peroxydase, commercialisé par | |
| CD 2 | ST 1 | BIOSYS | ST 11 | BIOSYS |
| CD 25 | ST 11 | BIOSYS | IOT 14 | IMMUNOTECH |

Les valeurs des densités optiques mesurées à 450 nm pour 25.10³ cellules mononucléées mises en oeuvre sont reportées dans le tableau 4 ci-dessous et comparent les mêmes cellules sans stimulation par la PHA et après 3 jours de stimulation.

**TABLEAU 4**

| | Densités optiques | | |
|---|---|---|---|
| Antigène | Cellules non activées | Cellules activées 3 jours | Blanc de réactifs |
| CD 25 | 0,064 | 0,529 | 0,044 |
| CD 2 | 0,129 | 0,768 | 0,027 |

Ces résultats montrent la croissance considérable des signaux spécifiques des 2 antigènes examinés, lorsque l'activation se produit.

### Exemple 7

dosage des antigènes CD 22 et HLA-Dr (ou HLA de classe II) présents sur les lymphocytes B.

L'exemple présent décrit le dosage de ces antigènes sur des cellules de la lignée RAJI qui est une lignée lymphoïde B humaine décrite par PULVERTAFT dans J. Clin. Pathol., 1965, 18, 261-274.

Pour l'immunocapture des cellules, on a adsorbé dans les puits de dosage, comme indiqué à l'exemple 1, soit l'anticorps S-classe II (BIOSYS) pour le dosage des antigènes CD22, soit l'anticorps SB4 (BIOSYS) pour le dosage des antigènes HLA de classe II (ou HLA-Dr).

### a) Dosage de l'antigène CD22 :

L'anticorps SB22 (BIOSYS) a été marqué à l'iode 125 selon la méthode décrite dans l'exemple 3. Le dosage des antigènes CD22 est réalisé de la manière indiquée à l'exemple 3 pour les antigènes CD4 et CD8, en introduisant dans les micropuits successivement : 5.10⁴ cellules de la lignée RAJI puis 10⁵ cpm de l'anticorps SB 22 marqué. Après un délai d'une heure à 4°C, les micropuits sont vidés par retournement de la plaque puis lavés 4 fois par 200 µl de PBS par puits à chacun des lavages. La radioactivité fixée aux cellules est récupérée et comptée comme dans l'exemple 3. On obtient ainsi un comptage de 760 dpm pour le puits contenant 5 x 10⁴ cellules RAJI avec un blanc de réactifs (sans cellules) de 50 dpm.

### b) Dosage de l'antigène HLA-Dr :

On introduit successivement dans les puits 10⁵ cellules et le conjugué anticorps S-classe II marqué à la peroxydase selon le procédé décrit à l'exemple 1. Le dosage est réalisé selon le même protocole que dans l'exemple 1. On obtient ainsi une densité optique de 1,840 et un blanc de réactifs sans cellules de 0,105.

### Exemple 8

Utilisation du procédé selon l'invention pour la mesure d'antigènes portés par les lymphocytes T et les lymphocytes B du sang humain.

### A) Préparation des plaques :

On utilise des plaques de microtitration préparées comme à l'exemple 1, excepté que,dans chaque puits de la microplaque, on adsorbe à la fois des anticorps monoclonaux capables de fixer au moins la totalité des lymphocytes T (s classe I de BIOSYS) et des lymphocytes B (S classe II de BIOSYS), chacun étant utilisé à la concentration de 5 µg/ml.

### B) Dosage immunométrique :

Les cellules mononucléées sont séparées du sang total à l'aide de FICOLL® dans les conditions de l'exemple 1 ou de l'exemple 5, puis on introduit 80 000 cellules mononucléées par puits dans 75 µl de tampon PBS. Les lymphocytes T sont mesurés dans certains puits avec l'anticorps ST 11 (anti-CD 2) de BIOSYS ; les lymphocytes B sont mesurés dans d'autres puits avec l'anticorps SB 3 (anti-CD 37) de BYOSIS qui reconnaît tous les lymphocytes B périphériques. Ces anticorps sont préalablement couplés à la peroxydase selon le protocole décrit à l'exemple 1. Le dosage proprement dit est conduit comme à l'exemple 1.

### C) Résultats :

Les nombres absolus de lymphocytes T et de lymphocytes B contenus dans l'échantillon examiné sont déterminés en se rapportant à des courbes d'étalonnage établies en traitant identiquement des cellules mononucléées dans lesquelles les lymphocytes T et B ont été dénombrés par une technique de référence.

Alternativement, grâce à des étalons appropriés, on peut aussi exprimer les résultats en concentrations molaires des antigènes CD2 et CD37 dans l'échantillon examiné.

### Exemple 9

Dosage des antigènes GpIIb-IIIa et CD 9 des plaquettes sanguines humaines.

Ce dosage est effectué selon l'exemple 3 en utilisant des anticorps monoclonaux radiomarqués à l'iode 125.

Les plaquettes sont isolées du sang humain par centrifugation en présence de liquide de perfusion PLASMION® (laboratoire R. Bellon). On mélange dans un tube 5 ml de sang plus 5 ml de tampon phosphate salin (PBS) et 10 ml de PLASMION®. Le tube est alors centrifugé pendant 10 minutes à 1 500 tr/min. Les plaquettes rassemblées dans le surnageant sont prélevées par aspiration à la pipette. Elles sont lavées une fois dans du PBS en mélangeant 1 volume de la suspension plaquettaire avec 10 ml de PBS, puis centrifugées pendant 10 minutes à 1000 x g. Finalement, on recueille le culot plaquettaire qui est remis en suspension dans du PBS (2 ml). Les plaquettes sont comptées et leur concentration ajustée à 2,5 millions/ml de PBS.

Pour doser l'antigène GpIIb-IIIa, on effectue l'immunocapture des plaquettes par l'anticorps monoclonal IOB2 (Immunotech) et le marquage de l'antigène GpIIb-III a par l'anticorps monoclonal P2 (Immunotech).

Pour doser l'antigène CD 9, on effectue l'immunocapture par l'anticorps monoclonal P2 et le marquage par l'anticorps monoclonal IOB2. On mesure le nombre de dpm pour un échantillon comprenant 200 000 plaquettes.

Les résultats sont portés dans le tableau 5.

**TABLEAU 5**

| Antigène mesuré | Echantillon mesuré (dpm) | Témoin sans cellules (dpm) |
|---|---|---|
| CD 9 | 1 927 | 113 |
| GpIIb-IIIa | 2 891 | 238 |

### Exemple 10

Evaluation du phénotype d'une leucémie par le procédé immunoenzymométrique.

Le phénotypage des cellules tumorales d'un patient leucémique est un examen de diagnostic systématique réalisé pour caractériser le type et l'origine des cellules leucémiques du patient (T, B, granulocytes, myéloblastes, etc.). Cet examen est classiquement effectué par la technique d'immunofluorescence en utilisant une batterie d'anticorps monoclonaux et en observant et dénombrant les cellules positives au microscope. On utilise également la cytométrie en flux pour analyser le marquage des cellules.

L'emploi d'anticorps monoclonaux par le procédé selon l'invention permet d'identifier les grands groupes cliniquement importants de leucémies aiguës ou chroniques en apportant en outre une information quantitative sur les densités relatives des différents antigènes examinés.

Dans cet exemple on a préparé 6 anticorps monoclonaux, couplés à la peroxydase, spécifique d'antigènes présents sur les cellules leucémiques :
- ST 1 et ST 11 (BIOSYS) : anti-CDE 5 et anti-CD 2
- SB 3 (BIOSYS) : anti-CD 37
- S-CALLA (SANOFI) : anti-CALLA (ou anti-CD10)
- S-classe II (BIOSYS) : anti-HLA-Dr (ou anti-HLA de classe II)
- SMY 15 (BIOSYS) : anti-CD 15

La plaque de microtitration est préparée par adsorption préalable d'un mélange d'anticorps monoclonaux anti-CD 45 (S-LCA, BIOSYS) et anti-HLA de classe I (S-classe I, BIOSYS).

En utilisant les 6 anticorps monoclonaux sélectionnés ci-dessus, on a évalué le phénotype d'une leucémie lymphoïde chronique B (LLC-B) qui a par ailleurs été caractérisée par la méthode conventionnelle.

Pour 80 000 cellules mononucléées totales introduites par puits, ou pour les puits témoins sans cellules, les densités optiques mesurées à 492 nm sont reportées dans le tableau 6 ci-dessous. Pour le puits témoin, comportant les cellules mais aucun anticorps marqué à la peroxydase, la densité optique est 0,110.

**TABLEAU 6**

| Anticorps monoclonal et antigène correspondant | Densité optique | |
|---|---|---|
| | Echantillon avec cellules | Témoin sans cellules |
| ST 11 (CD2) | 0,270 | 0,030 |
| ST 1 (CD5) | 1,350 | 0,014 |
| SB 3 (CD37) | 0,850 | 0,005 |
| S-classe II (HLA-Dr) | 1,640 | 0,010 |
| SMY 15 (CD15) | 0,235 | 0,007 |
| CALLA (CD10) | 0,160 | 0,002 |

Ainsi, les cellules leucémiques étudiées portent abondamment les antigènes CD5, CD37 et HLA de classe II, mais elles ne portent pas ou très faiblement les antigènes CD15, CALLA et CD2, ce qui est caractéristique d'une LLC-B.

## Revendications

1. Trousse pour le dosage immunométrique de plusieurs antigènes de surface caractéristiques chacun d'une sous-population cellulaire, lesdites sous-populations étant constitutives d'une population, ladite trousse comprenant comme composants :
a) un support solide sur lequel sont fixés par liaison convalente ou par adsorption physique un ou plusieurs anticorps monoclonaux dirigés contre des antigènes de surface communs à toute ladite population cellulaire, autres que les antigènes caractéristiques desdits sous-populations;
b) plusieurs solutions contenant chacune un anticorps monoclonal spécifique chacun dudit antigène caractéristique d'une sous-population cellulaire à doser, marqué par une sonde radioactive ou une sonde enzymatique;
c) dans le cas d'anticorps marqués par une sonde enzymatique, une ou plusieurs solutions apportant les réactifs nécessaires pour révéler l'activité de l'enzyme;
d) éventuellement, un composant additionnel constitué par une solution tampon de lavage et/ou des échantillons permettant l'étalonnage et le contrôle de qualité du dosage.

2. Trousse selon la revendication 1, dans laquelle le composant a) est un support solide constitué par une plaque de microtitration dans les puits de laquelle sont fixés le ou les anticorps destinés à immobiliser les cellules de ladite population.

3. Trousse selon la revendication 1, dans laquelle le composant (a) est un support magnétique particulaire.

4. Trousse selon l'une quelconque des revendications 1 à 3, dans laquelle le composant (a) est constitué d'un support solide sur lequel sont fixés un ou plusieurs anticorps monoclonaux HLA de classe I.

5. Trousse selon l'une quelconque des revendications 1 à 4, dans laquelle les anticorps monoclonaux du composant (b) sont marqués par une sonde radio-isotopique à l'iode 125 ou à l'iode 131.

6. Trousse selon l'une quelconque des revendications 1 à 4, dans laquelle les anticorps monoclonaux du composant (b) sont marqués par une sonde enzymatique.

7. Trousse selon la revendication 6, dans laquelle les anticorps monoclonaux du composant (b) sont marqués à la peroxydase et le composant (c) comprend essentiellement de l'eau oxygénée et un chromogène choisi parmi l'orthophénylènediamine, l'acide azino-2,2' bis (éthyl-3 benzothiazoline sulfonique-6), la diamino-3,3' benzidine, l'amino-3 éthyl-9 carbazole ou l'un de leurs sels solubles dans l'eau.

8. Trousse selon la revendication 6, dans laquelle les anticorps monoclonaux du composant (b) sont marqués à la phosphatase alcaline et le composant (c) est constitué par le phosphate de paranitrophényle, par le phosphate de méthyl-4 umbelliféryle ou par le phosphate de bromo-5 chloro-4 indole-3 yle.

9. Trousse selon la revendication 6, dans laquelle les anticorps monoclonaux du composant (b) sont marqués à la β-galactosidase et le composant (c) est constitué par l'orthonitrophényl β-D-galactopyranoside ou le méthyl-4 umbelliféryl β-D-galactopyranoside.

10. Trousse selon la revendication 6, dans laquelle les anticorps monoclonaux du composant (b) sont marqués à l'acétylcholinestérase et le composant (c) comprend essentiellement de l'acétylthiocholine et de l'acide dithio-5,5' nitro-2 benzoïque ou un de leurs sels solubles dans l'eau.

11. Trousse selon la revendication 1, pour le dosage de plusieurs antigènes de surface des éléments figurés du sang humain tels que les leucocytes, les lymphocytes, les lymphocytes T, les lymphocytes B, les granulocytes et les plaquettes.

12. Trousse selon la revendication 11, pour le dosage des antigènes CD4 et CD8 des lymphocytes T humains porteurs de l'un ou l'autre de ces antigènes, appelés lymphocytes T4 et lymphocytes T8.

13. Trousse selon la revendication 1, pour le dosage de plusieurs antigènes de surface d'un microorganisme pathogène.

14. Trousse selon la revendication 13, dans laquelle le microorganisme pathogène est Candida albicans.

15. Trousse selon la revendication 1, pour le dosage de plusieurs antigènes des cellules tumorales.

16. Trousse selon la revendication 15, dans laquelle les cellules tumorales sont celles des cancers de l'appareil urinaire ou celles des hémopathies malignes.

17. Trousse selon la revendication 1, pour le dosage d'antigènes spécifiques des sous-populations lymphocytaires T, T4, T8 et B.

18. Trousse selon la revendication 1, pour la caractérisation et le dosage de différents antigènes constituant l'équipement antigénique de surface des cellules tumorales d'hémopathies malignes.

19. Procédé de dosage immunométrique de plusieurs antigènes de surface caractéristiques chacun d'une sous-population cellulaire, lesdites sous-populations étant constitutives d'une population, caractérisé en ce qu'il consiste :
a) à immobiliser ladite population cellulaire sur un support solide par un ou plusieurs anticorps monoclonaux, préalablement fixés par liaison covalente ou par adsorption physique sur ledit support et qui sont spécifiques d'antigènes de surface de cette population autre que les antigènes caractéristiques desdites sous-populations et, dans la même étape, à marquer directement des sous-populations cellulaires constitutives de la population fixée au moyen de plusieurs solutions contenant chacune un anticorps monoclonal spécifique chacun d'un antigène de surface de l'une des sous-populations cellulaires à doser, lesdits anticorps portant une sonde radio-isotopique ou enzymatique;
b) à observer une période d'incubation;
c) à laver le support pour éliminer les cellules non immobilisées et l'excès d'anticorps
d) dans le cas où l'anticorps porte une sonde enzymatique, à ajouter le ou les réactifs nécessaires (substrat et chromogène) pour révéler l'activité de l'enzyme et,
e) à lire les résultats, soit par comptage de la radioactivité, soit par mesure des signaux lumineux (coloration ou fluorescence) en se référant éventuellement à une gamme d'étalonnage.

20. Procédé de dosage selon la revendication 19, dans lequel le support solide est tel que défini dans l'une des revendications 2 ou 3.

21. Procédé de dosage selon l'une des revendications 19 ou 20 dans lequel les anticorps monoclonaux destinés au marquage portent une sonde enzymatique.

22. Procédé de dosage selon la revendication 21, dans lequel la sonde enzymatique est la peroxydase et l'activité de l'enzyme est révélée par l'eau oxygénée et un chromogène choisi parmi l'orthophénylène diamine, l'acide azino-2,2' bis (éthyl-3 benzothiazoline sulfonique-6), la diamino-3,3' benzidine, l'amino-3 éthyl-9 carbazole ou l'un de leurs sels solubles dans l'eau.

23. Procédé de dosage selon la revendication 21, dans lequel la sonde enzymatique est la phosphatase alcaline et l'activité de l'enzyme est révélée par le phosphate de paranitrophényle, le phosphate de méthyl-4 umbelliféryle ou par le phosphate de bromo-5 chloro-4 indole-3 yle.

24. Procédé de dosage selon la revendication 21, dans lequel la sonde enzymatique est la β-galactosidase et l'activité de l'enzyme est révélée par l'orthonitrophényl β-D-galactopyranoside ou le méthyl-4 umbeiliféryl β-D-galactopyranoside.

25. Procédé de dosage selon la revendication 21, dans lequel la sonde enzymatique est l'acétylcholinestérase et l'activité de l'enzyme est révélée par l'acétylthiocholine et, comme chromogène, l'acide dithio-5,5' nitro-2 benzoïque ou l'un de leurs sels solubles dans l'eau.

26. Procédé de dosage selon la revendication 19 ou 20, dans lequel les anticorps monoclonaux destinés au marquage portent une sonde radio-isotopique à l'iode 125 ou à l'iode 131.

27. Procédé selon l'une quelconque des revendications 19 ou 20, dans lequel la durée totale d'exécution du dosage est inférieure ou égale à 1 heure.

28. Procédé selon la revendication 19, dans lequel le ou les anticorps monoclonaux fixés sur le support solide sont des anticorps HLA de classe I.

29. Procédé selon la revendication 19, pour le dosage de plusieurs antigènes de surface des éléments figurés du sang humain.

30. Procédé selon la revendiction 29, dans lequel les éléments figurés du sang humain sont les leucocytes, les lymphocytes, les lymphocytes T, les lymphocytes T porteurs du marqueur CD4 appelés lymphocytes T4, les lymphocytes T porteurs du marqueur CD8 appelés lymphocytes T8, les lymphocytes B, les granulocytes, les plaquettes.

31. Procédé selon la revendication 19, pour le dosage de plusieurs antigènes de surface d'un microorganisme pathogène.

32. Procédé selon la revendication 31, dans lequel le microorganisme pathogène est Candida albicans.

33. Procédé selon la revendication 19, pour le dosage de plusieurs des antigènes membranaires des cellules tumorales.

34. Procédé selon la revendication 33, dans lequel les cellules tumorales sont celles des cancers de l'appareil urinaire ou celles des hémopathies malignes.

35. Procédé selon la revendication 19, pour le dosage d'antigènes spécifiques des sous-populations lymphocytaires T, T4, T8 et B.

36. Procédé selon la revendication 19 pour la caractérisation et le dosage de différents antigènes constituant l'équipement de surface de cellules tumorales d'hémopathies malignes.

## Claims

1. Kit for the immunometric assay of several surface antigens each one being characteristic of a cell subpopulation, said subpopulations constituting a population, said kit comprising as components:
a) a solid support on which one or more monoclonal antibodies are fixed by covalent bonding or physical adsorption, the said monoclonal antibodies being directed against surface antigens common to the whole said cell population, other than said antigens characteristic of said subpopulations;
b) several solutions each containing a monoclonal antibody specific for said antigen characteristic of a cell subpopulation to be assayed, which is labeled with a radioactive probe or an enzyme probe;
c) in the case of antibodies labeled with an enzyme probe, one or more solutions providing the reagents necessary for developing the activity of the enzyme;
d) if appropriate, an additional component consisting of a washing buffer solution and/or samples permitting standardization and quality control of the assay.

2. Kit according to claim 1, in which component (a) is a solid support consisting of a microtiter plate in whose wells the antibody or antibodies for immobilization of the cells of said population are fixed.

3. Kit according to claim 1, in which component (a) is a particulate magnetic support.

4. Kit according to any one of claims 1 to 3, in which component (a) consists of a solid support to which one or more class I HLA monoclonal antibodies are fixed.

5. Kit according to any one of claims 1 to 4, in which the monoclonal antibodies of component (b) are labeled with an iodine 125 or iodine 131 radioisotopic probe.

6. Kit according to any one of claims 1 to 4, in which the monoclonal antibodies of component (b) are labeled with an enzyme probe.

7. Kit according to claim 6, in which the monoclonal antibodies of component (b) are labeled with peroxidase and component (c) essentially comprises hydrogen peroxide and a chromogen selected from orthophenylenediamine, 2,2'-azino-bis(3-ethyl-6-benzothiazoline sulfonic) acid, 3,3'-diaminobenzidine, 3-amino-9-ethylcarbazole or one of their water-soluble salts.

8. Kit according to claim 6, in which the monoclonal antibodies of component (b) are labeled with alkaline phosphatase and in which component (c) consists of paranitrophenyl phosphate, 4-methylumbelliferyl phosphate or 5-bromo-4-chloroindol-3-yl phosphate.

9. Kit according to claim 6, in which the monoclonal antibodies of component (b) are labeled with betagalactosidase and in which component (c) consists of orthonitrophenyl beta-D-galactopyranoside or 4-methylumbelliferyl beta-D-galactopyranoside.

10. Kit according to claim 6, in which the monoclonal antibodies of component (b) are labeled with acetylcholinesterase and in which component (c) essentially comprises acetylthiocholine and 5,5'-dithio-2-nitrobenzoic acid or one of their water-soluble salts.

11. Kit according to claim 1, for the assay of several surface antigens of the formed elements of human blood, such as the leukocytes, the lymphocytes, the T lymphocytes, the B lymphocytes, the granulocytes and the platelets.

12. Kit according to claim 11, for the assay of the CD4 or CD8 antigens of the human T lymphocytes carrying this antigen, which are called T4 lymphocytes or T8 lymphocytes.

13. Kit according to claim 1, for the assay of several surface antigens of a pathogenic microorganism.

14. Kit according to claim 13, in which the pathogenic microorganism is Candida albicans.

15. Kit according to claim 1, for the assay of several antigens of tumoral cells.

16. Kit according to claim 15, in which the tumoral cells are those of cancers of the urinary system or those of malignant hemopathies.

17. Kit according to claim 1, for the assay of specific antigens of T, T4, T8 and B lymphocytic subpopulations.

18. Kit according to claim 1, for the characterization and assay of different antigens constituting the surface antigenic equipment of the tumoral cells of malignant hemopathies.

19. Process for the immunometric assay of several surface antigens each one being characteristic of a cell subpopulation, said subpopulations constituting one population, characterized in that it consists in:
a) immobilizing said cell population on a solid support by using one or more monoclonal antibodies, previously fixed to the said support by covalent bonding or by physical adsorption and which are specific of surface antigens of said population, other than the antigens characteristic of said subpopulations and, in the same step, directly labeling the cell subpopulations constituting the fixed population by means of several solutions each one containing a monoclonal antibody specific of a surface antigen of one of the cell subpopulations to be assayed, said antibodies carrying a radioisotopic or enzyme probe;
b) observing an incubation period;
c) washing the support to remove the non-immobilized cells and the excess antibody;
d) in the case where the antibody carries an enzyme probe, adding the reagent or reagents (substrate and chromogen) necessary for developing the activity of the enzyme; and
e) reading the results either by counting the radioactivity or by measuring the light signals (coloration or fluorescence) with reference to a standard range if appropriate.

20. Assay process according to claim 19, in which the solid support is as defined in claim 2 or 3.

21. Assay process according to one of claims 19 or 20, in which the monoclonal antibodies intended for labeling carry an enzyme probe.

22. Assay process according to claim 21, in which the enzyme probe is peroxidase and the activity of the enzyme is developed by hydrogen peroxide and a chromogen selected from orthophenylenediamine, 2,2'azino-bis(3-ethyl-6-benzothiazoline sulfonic) acid, 3,3'-diaminobenzidine, 3-amino-9-ethylcarbazole or one of their water-soluble salts.

23. Assay process according to claim 21, in which the enzyme probe is alkaline phosphatase and the activity of the enzyme is developed by para-nitrophenyl phosphate, 4-methylumbelliferyl phosphate or 5-bromo-4-chloroindol-3-yl phosphate.

24. Assay process according to claim 21, in which the enzyme probe is beta-galactosidase and
the activity of the enzyme is developed by ortho-nitrophenyl beta-D-galactopyranoside or 4-methylumbelliferyl beta-D-galactopyranoside.

25. Assay process according to claim 21 in which the enzyme probe is acetylcholinesterase and the activity of the enzyme is developed by acetylthiocholine and, as the chromogen, 5,5'-dithio-2-nitrobenzoic acid or one of their water-soluble salts.

26. Assay process according to claim 19 or 20, in which the monoclonal antibodies intended for labeling carry an iodine 125 or iodine 131 radioisotopic probe.

27. Process according to claim 19 or 20, in which the total time required to carry out the assay is less than or equal to 1 hour.

28. Process according to claim 19, in which the monoclonal antibody or antibodies fixed to the solid support are class I HLA antibodies.

29. Process according to claim 19, for the assay of one or more surface antigens of the formed elements of human blood.

30. Process according to claim 29, in which the formed elements of human blood are the leukocytes, the lymphocytes, the T lymphocytes, the T lymphocytes carrying the CD4 marker, called T4 lymphocytes, the T lymphocytes carrying the CD8 marker, called T8 lymphcytes, the B lymphocytes, the granulocytes and the platelets.

31. Process according to claim 19, for the assay of several of the surface antigens of a pathogenic microorganism.

32. Process according to claim 31 in which the pathogenic microorganism is Candida albicans.

33. Process according to claim 19, for the assay of several of the membrane antigens of tumoral cells.

34. Process according to claim 33, in which the tumoral cells are cells of cancers of the urinary systems or cells of malignant hemopathies.

35. Process according to claim 19, for the assay of antigens specific of T, T4, T8 and B lymphocytic subpopulations.

36. Process according to claim 19 for the characterization and assay of different antigens constituting the surface equipment of tumoral cells of malignant hemopathies.

## Patentansprüche

1. Kit zur immunometrischen Bestimmung mehrerer Oberflächenantigene, die jeweils für eine zelluläre Unterpopulation charakteristisch sind, welche Unterpopulationen eine Population ausmachen, welcher Kit folgende Bestandteile aufweist:
a) einen festen Träger, auf dem durch kovalente Bindung oder durch physikalische Adsorption ein oder mehrere monoklonale Antikörper fixiert sind, die gegen der gesamten Zellpopulation gemeinsame Oberflächenantigene und nicht gegen die für die Unterpopulationen charakteristischen Antigene gerichtet sind;
b) mehrere Lösungen, die jeweils einen monoklonalen Antikörper enthalten, von denen jeder spezifisch für das zu bestimmende und für eine zelluläre Unterpopulation charakteristische Antigen ist, welcher Antikörper mit einer radioaktiven Sonde oder einer enzymatischen Sonde markiert ist ;
c) im Fall von mit einer enzymatischen Sonde markierten Antikörpern eine oder mehrere die zur Anzeige der Aktivität des Enzyms notwendigen Reagenzien einführende Lösung(en);
d) gegebenenfalls einen zusätzlichen Bestandteil, bestehend aus einer Waschpufferlösung und/oder Proben, die die Kalibrierung und die Qualitätskontrolle der Bestimmung gestatten.

2. Kit nach Anspruch 1, worin der Bestandteil a) ein fester Träger ist, welcher durch eine Mikrotiterplatte gebildet ist, in deren Vertiefungen der oder die zur Immobilisierung der Zellen der Population bestimmte(n) Antikörper fixiert ist/sind.

3. Kit nach Anspruch 1, worin der Bestandteil a) ein partikelförmiger Magnetträger ist.

4. Kit nach einem der Ansprüche 1 bis 3, worin der Bestandteil a) durch einen festen Träger gebildet ist, auf dem ein oder mehrere monoklonale HLA-Antikörper der Klasse I fixiert sind.

5. Kit nach einem der Ansprüche 1 bis 4, worin die monoklonalen Antikörper des Bestandteils b) mit einer radioisotopen Jod 125- oder Jod 131-Sonde markiert sind.

6. Kit nach einem der Ansprüche 1 bis 4, worin die monoklonalen Antikörper des Bestandteils b) mit einer enzymatischen Sonde markiert sind.

7. Kit nach Anspruch 6, worin die monoklonalen Antikörper des Bestandteils b) mit Peroxidase markiert sind und der Bestandteil c) im wesentlichen Wasserstoffperoxid und ein Chromogen, ausgewählt aus Orthophenylendiamin, 2,2'-Azino-bis-(3-ethyl-6-benzothiazolinsulfon)-säure, 3,3'-Diaminobenzidin, 3-Amino-9-ethylcarbazol oder einem wasserlöslichen Salz davon, umfaßt.

8. Kit nach Anspruch 6, worin die monoklonalen Antikörper des Bestandteils b) mit alkalischer Phosphatase markiert sind und der Bestandteil c) durch Paranitrophenylphosphat, 4-Methylumbelliferylphosphat oder 5-Brom-4-chlor-indol-3-yl-phosphat gebildet ist.

9. Kit nach Anspruch 6, worin die monoklonalen Antikörper des Bestandteils b) mit β-Galactosidase markiert sind und der Bestandteil c) durch Orthonitrophenyl-β-D-galactopyranosid oder 4-Methyl-umbelliferyl-β-D-galactopyranosid gebildet ist.

10. Kit nach Anspruch 6, worin die monoklonalen Antikörper des Bestandteils b) mit Acetylcholinesterase markiert sind und der Bestandteil c) im wesentlichen Acetylthiocholin und 5,5'-Dithio-2-nitro-benzoesäure oder eines der wasserlöslichen Salze davon, umfaßt.

11. Kit nach Anspruch 1 zur Bestimmung mehrerer Oberflächenantigene der im menschlichen Blut vorhandenen Elemente, wie Leukozyten, Lymphozyten, T-Lymphozyten, B-Lymphozyten, Granulozyten und Plättchen.

12. Kit nach Anspruch 11 zur Bestimmung der CD4- und CD8-Antigene der menschlichen T-Lymphozyten als Träger des einen oder anderen dieser Antigene, genannt T4-Lymphozyten und T8-Lymphozyten.

13. Kit nach Anspruch 1 zur Bestimmung mehrerer Oberflächenantigene eines pathogenen Mikroorganismus.

14. Kit nach Anspruch 13, worin der pathogene Mikroorganismus Candida albicans ist.

15. Kit nach Anspruch 1 zur Bestimmung mehrerer Antigene von Tumorzellen.

16. Kit nach Anspruch 15, worin die Tumorzellen Krebszellen des Harnapparats oder solche maligner Hämopathien sind.

17. Kit nach Anspruch 1 zur Bestimmung von spezifischen Antigenen der Lymphozyten-Unterpopulationen T, T4, T8 und B.

18. Kit nach Anspruch 1 zur Charakterisierung und Bestimmung verschiedener Antigene, die die Antigenausrüstung der Oberfläche von Tumorzellen maligner Hämopathien bilden.

19. Verfahren zur immunometrischen Bestimmung mehrerer Oberflächenantigene, die jeweils für eine zelluläre Unterpopulation charakteristisch sind, welche Unterpopulationen eine Population ausmachen, dadurch gekennzeichnet, daß es darin besteht:
a) die Zellpopulation auf einem festen Träger mittels eines oder mehrere monoklonaler Antikörper(s), die zuvor durch kovalente Bindung oder durch physikalische Adsorption auf dem Träger fixiert wurden und spezifisch für andere Oberflächenantigene dieser Population als für die für die Unterpopulationen charakteristischen Antigene sind, zu immobilisieren und in derselben Stufe die fixierte Population bildende zelluläre Unterpopulationen direkt mit mehreren Lösungen zu markieren, welche jeweils einen monoklonalen Antikörper enthalten, von denen jeder spezifisch für ein zu bestimmendes Oberflächenantigen einer der zellulären Unterpopulationen ist, wobei die Antikörper mit einer radioisotopen oder enzymatischen Sonde versehen sind;
b) einen Inkubationszeitraum einzuhalten;
c) den Träger zur Entfernung der nicht immobilisierten Zellen und des Antikörperüberschusses zu waschen;
d) für den Fall, daß der Antikörper eine enzymatische Sonde aufweist, das oder die zur Anzeige der Aktivität des Enzyms notwendige(n) Reagens/zien (Substrat und Chromogen) zuzugeben, und
e) die Resultate entweder durch Zählung der Radioaktivität oder durch Messung der Lichtsignale (Färbung oder Fluoreszenz), gegebenenfalls unter Bezugnahme auf eine Kalibrierskala, abzulesen.

20. Bestimmungsverfahren nach Anspruch 19, worin der feste Träger wie in einem der Ansprüche 2 oder 3 definiert ist.

21. Bestimmungsverfahren nach einem der Ansprüche 19 oder 20, worin die zur Markierung bestimmten monoklonalen Antikörper mit einer enzymatischen Sonde versehen sind.

22. Bestimmungsverfahren nach Anspruch 21, worin die enzymatische Sonde Peroxidase ist und die Aktivität des Enzyms durch Wasserstoffperoxid und ein Chromogen, ausgewählt aus Orthophenylendiamin, 2,2'-Azino-bis-(3-ethyl-6-benzothiazolinsulfon)-säure, 3,3'-Diaminobenzidin, 3-Amino-9-ethylcarbazol oder einem wasserlöslichen Salz davon, angezeigt wird.

23. Bestimmungsverfahren nach Anspruch 21, worin die enzymatische Sonde alkalische Phosphatase ist und die Aktivität des Enzyms durch Paranitrophenylphosphat, 4-Methylumbelliferylphosphat oder 5-Brom-4-chlor-indol-3-yl-phosphat angezeigt wird.

24. Bestimmungsverfahren nach Anspruch 21, worin die enzymatische Sonde β-Galactosidase ist und die Aktivität des Enzyms durch Orthonitrophenyl-β-D-galactopyranosid oder 4-Methyl-umbelliferyl-β-D-galactopyranosid angezeigt wird.

25. Bestimmungsverfahren nach Anspruch 21, worin die enzymatische Sonde Acetylcholinesterase ist und die Aktivität des Enzyms durch Acetylthiocholin und, als Chromogen, 5,5'-Dithio-2-nitro-benzoesäure oder eines der wasserlöslichen Salze davon angezeigt wird.

26. Bestimmungsverfahren nach Anspruch 19 oder 20, worin die zur Markierung bestimmten monoklonalen Antikörper mit einer radioisotopen Jod 125- oder Jod 131-Sonde versehen sind.

27. Verfahren nach einem der Ansprüche 19 oder 20, worin die Gesamtdauer der Durchführung der Bestimmung unter oder gleich 1 Stunde beträgt.

28. Verfahren nach Anspruch 19, worin der oder die auf dem festen Träger fixierte(n) monoklonale(n) Antikörper HLA-Antikörper der Klasse I sind.

29. Verfahren nach Anspruch 19 zur Bestimmung mehrerer Oberflächenantigene der im menschlichen Blut vorhandenen Elemente.

30. Verfahren nach Anspruch 29, worin die im menschlichen Blut vorhandenen Elemente Leukozyten, Lymphozyten, T-Lymphozyten, T-Lymphozyten als Träger des Markers CD4, genannt T4-Lymphozyten, T-Lymphozyten als Träger des Markers CD8, genannt T8-Lymphozyten, B-Lymphozyten, Granulozyten und Plättchen sind.

31. Verfahren nach Anspruch 19 zur Bestimmung mehrerer Oberflächenantigene eines pathogenen Mikroorganismus.

32. Verfahren nach Anspruch 31, worin der pathogene Mikroorganismus Candida albicans ist.

33. Verfahren nach Anspruch 19 zur Bestimmung mehrerer Membranantigene von Tumorzellen.

34. Verfahren nach Anspruch 33, worin die Tumorzellen Krebszellen des Harnapparats oder solche maligner Hämopathien sind.

35. Verfahren nach Anspruch 19 zur Bestimmung von spezifischen Antigenen der Lymphozyten-Unterpopulationen T, T4, T8 und B.

36. Verfahren nach Anspruch 19 zur Charakterisierung und Bestimmung verschiedener Antigene, die die Antigenausrüstung der Oberfläche von Tumorzellen maligner Hämopathien bilden.
